# EUROPEAN PATENT APPLICATION

(11) **EP 4 785 889 A1**
(43) Date of publication of application: **05.08.2026**
(21) Application number: 26156180.7
(22) Date of filing: 03.02.2026
(51) Int. Cl.: A61B 34/00, A61B 90/00, A61G 13/04, A61B 34/30

(54) **A SYSTEM FOR MOVING AN OPERATING TABLE DURING SURGERY**

(30) Priority: 03.02.2025 US 202563752957 P; 29.01.2026 US 202619463874
(71) Applicant: Baxter Medical Systems GmbH + Co. KG, 07318 Saalfeld (DE)
(72) Inventor: LÖSER, Steffen, Batesville, 47006 (US); MUECKNER, Michael, Batesville, 47006 (US); THI, Quynh Trang Nguyen, Batesville, 47006 (US); GOECKERITZ, Stefan, Batesville, 47006 (US)
(74) Representative: Reddie & Grose LLP

(57) **Abstract**

A surgical system (10) includes a user interface (14) and an operating table (16) with at least one robotic arm (18) supported on a pedestal (20) that can selectively tilt, based on instructions from the user interface (14), the operating table (16) about a longitudinal axis L(a) and a transverse axis T(a). A camera (21) is configured to capture image data (22) within a field-of-view ("FOV") of a patient operating region (24) and a display module (26) is configured to display the image data (22). A control system (100) is configured to receive a current tilt of the operating table (16) about the longitudinal axis L(a) and the transverse axis T(a), and graphically generate a gravity vector (28) corresponding to the current tilt over the image data (22) displayed on the display module (26).

## Description

### FIELD OF THE DISCLOSURE

The present disclosure generally relates to a system, method, and program for streamlining moving an operating table during surgery.

### BACKGROUND

In many types of surgeries, the use of gravity is utilized to move organs out of the way, and thus allow optimal access to a point of interest inside a patient's body. Robotic assistance has modernly been implemented in various surgical procedures for assisted and minimally invasive techniques. Typically, robotic arms are provided on a cart or other modular unit that is separated from an operating table. If a surgeon wants to tilt the operating table during surgery (e.g., to utilize gravity), a medical assistant has to remove instruments from the patient, relocate the patient by controlling movements of the operating table, and manually reinsert instruments into the patient's body. More recently, robotic arms have been coupled directly to the operating table, such that removal and reinsertion of the instruments are no longer always required as the robotic arms are moved with the operating table. The operating table, however, still needs to be controlled manually by the medical assistant, via instructions from the surgeon received verbally. Generally, the surgeon's view is defined by the field-of-view of a camera inside the patient, which is not necessarily aligned with the tilting axis of the operating room table. As a result, the limited field-of-view of the surgeon and having to interpret verbal instructions add additional steps and complications to surgical procedures.

Accordingly, the present disclosure provides a system, method, and program for streamlining moving an operating table during surgery.

### SUMMARY OF THE DISCLOSURE

According to one aspect of the present disclosure, a surgical system includes a user interface and an operating table with at least one robotic arm supported on a pedestal that can selectively tilt, based on instructions from the user interface, the operating table about a longitudinal axis and a transverse axis. A camera is configured to capture image data within a field-of-view ("FOV") of a patient operating region and a display module is configured to display the image data. A control system is configured to receive a current tilt of the operating table about the longitudinal axis and the transverse axis, and graphically generate a gravity vector corresponding to the current tilt over the image data displayed on the display module.

According to another aspect of the present disclosure, a surgical system includes a user interface and an operating table with at least one robotic arm supported on a pedestal that can selectively tilt, based on instructions from the user interface, the operating table about a longitudinal axis and a transverse axis. A camera is configured to capture image data within a field-of-view ("FOV") of a patient operating region and a display module is configured to display the image data. A control system is configured to receive a current tilt of the operating table about the longitudinal axis and the transverse axis, and graphically generate a coordinate diagram corresponding to a current tilt and an allowable remaining tilt over the image data displayed on the display module.

According to yet another aspect of the present disclosure, a computer program product for a surgical system includes a non-transitory computer-readable storage medium readable by one or more processing circuits and storing instructions for execution by one or more processors for performing a method of visually guiding, via a display module, a surgeon in tilting an operating table about a longitudinal axis and a transverse axis. The method includes the steps of receiving, from the operating table, a current tilt of the operating table about the longitudinal axis and the transverse axis, and graphically generating a gravity vector corresponding to the current tilt on the display module, and a marking positioned relative to the one or more virtual axes that corresponds to the current tilt.

According to still another aspect of the present disclosure, a method is provided for visually guiding, via a display module, a surgeon in tilting an operating table about a longitudinal axis and a transverse axis. The method includes receiving from the operating table, a current tilt of the operating table about the longitudinal axis and the transverse axis, and graphically generating a coordinate diagram corresponding to an allowable remaining tilt over the image data displayed on the display module.

These and other features, advantages, and objects of the present disclosure will be further understood and appreciated by those skilled in the art by reference to the following specification, claims, and appended drawings.

### BRIEF DESCRIPTION OF THE DRAWINGS

In the drawings:
FIG. 1 is a side elevational view of a surgical system within a medical environment, according to an aspect of the present disclosure;
FIG. 2 is a side perspective view of an operating table in a surgical system that can be selectively tilted, according to an aspect of the present disclosure;
FIG. 3 is a front view of a display module in a surgical system displaying image data from a camera, according to an aspect of the present disclosure;
FIG. 4A depicts a gravity vector and a coordinate diagram generated on a display module in a surgical system with a non-articulated operating table, according to an aspect of the present disclosure;
FIG. 4B depicts a gravity vector and a coordinate diagram generated on a display module in a surgical system with an articulated operating table and an endoscopic camera in a first position, according to an aspect of the present disclosure;
FIG. 4C depicts a gravity vector and a coordinate diagram generated on a display module in a surgical system with an articulated operating table and an endoscopic camera in a second position, according to an aspect of the present disclosure;
FIG. 5A is a front view of a display module of a first construction as a portable electronic device in a surgical system that is configured to display image data from a camera, according to an aspect of the present disclosure;
FIG. 5B is a front view of a display module of a second construction as an electronic computing device in a surgical system that is configured to display image data from a camera, according to an aspect of the present disclosure;
FIG. 5C is a perspective view of a display module of a third construction as a wearable device in a surgical system that is configured to display image data from a camera, according to an aspect of the present disclosure;
FIG. 6 is a schematic view of a control system of a surgical system, according to an aspect of the present disclosure;
FIG. 7 is a top plan view of a computer program that receives positional information from an operating table and overlays a visual indicator of the operating table position over image data on a display module, according to an aspect of the present disclosure; and
FIG. 8 is a flow chart of a method of providing visual indicators of positional information related to an operating table to a surgeon during a surgical operation, according to an aspect of the present disclosure.

### DETAILED DESCRIPTION

The present illustrated embodiments reside primarily in combinations of method steps and apparatus components related to a system, method, and program for streamlining moving an operating table during surgery. Accordingly, the apparatus components and method steps have been represented, where appropriate, by conventional symbols in the drawings, showing only those specific details that are pertinent to understanding the embodiments of the present disclosure so as not to obscure the disclosure with details that will be readily apparent to those of ordinary skill in the art having the benefit of the description herein. Further, like numerals in the description and drawings represent like elements.

The specific structures and processes illustrated in the attached drawings, and described in the following specification, are simply exemplary embodiments of the inventive concepts defined in the appended claims. Hence, specific dimensions and other physical characteristics relating to the embodiments disclosed herein are not to be considered as limiting, unless the claims expressly state otherwise.

The terms "including," "comprises," "comprising," or any other variation thereof, are intended to cover a non-exclusive inclusion, such that a process, method, article, or apparatus that comprises a list of elements does not include only those elements but may include other elements not expressly listed or inherent to such process, method, article, or apparatus. An element preceded by "comprises a . . . " does not, without more constraints, preclude the existence of additional identical elements in the process, method, article, or apparatus that comprises the element.

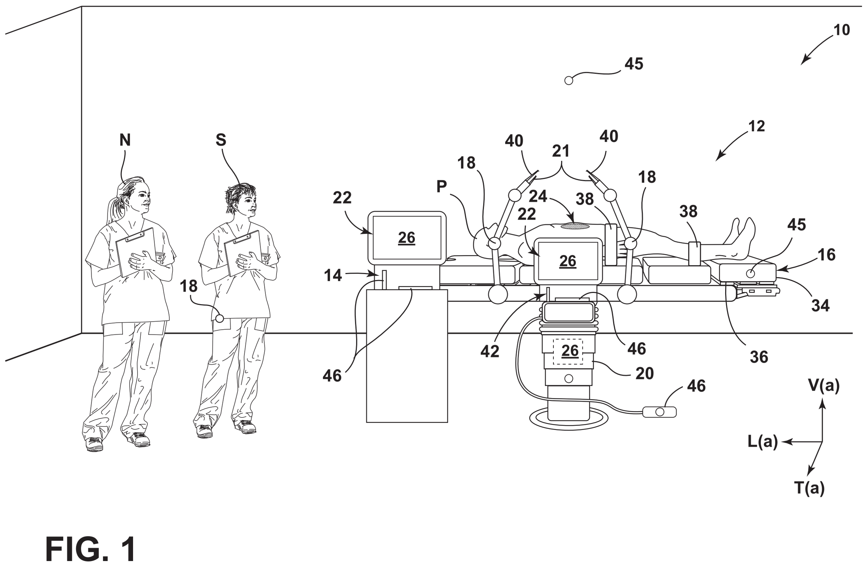

Referring initially to FIGS. 1-3, reference numeral 10 generally designates a surgical system for a medical environment 12. The surgical system 10 includes a user interface 14 and an operating table 16 with at least one robotic arm 18 supported on a pedestal 20 that can selectively tilt, based on instructions from the user interface 14, the operating table 16 about a longitudinal axis L(a) and a transverse axis T(a) that is transverse (e.g., perpendicular) to the longitudinal axis L(a). A camera 21 is configured to capture image data 22 within a field-of-view ("FOV") of a patient operating region 24 and a display module 26 is configured to display the image data 22. A control system 100 is configured to receive a current tilt of the operating table 16 about the longitudinal axis L(a) and the transverse axis T(a), and graphically generate at least one of a gravity vector 28 corresponding to the current tilt with respect to gravity over the image data 22 displayed on the display module 26 and/or a coordinate diagram 30 corresponding to a frame of reference of a current tilt and an allowable remaining tilt permitted by the user interface 14 over the image data 22 displayed on the display module 26.

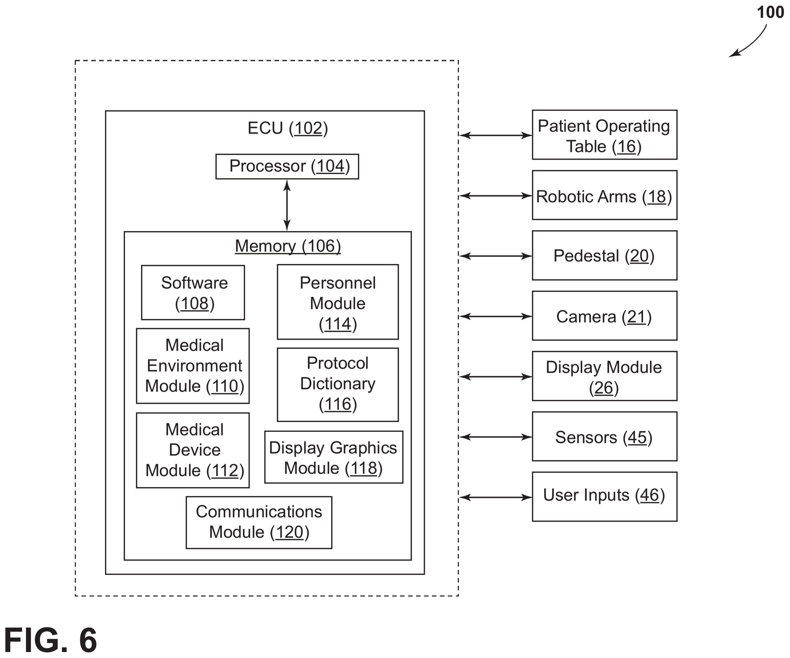

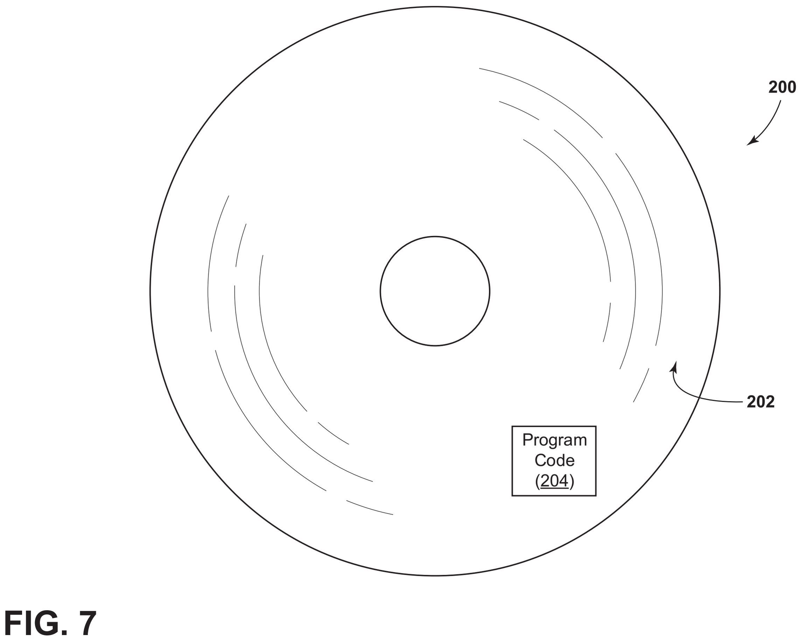

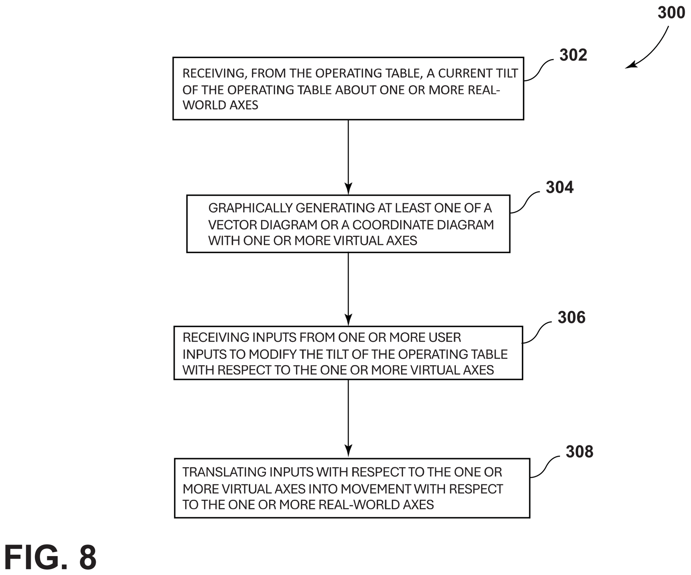

The surgical system 10 (FIG. 1), the control system 100 (FIG. 6), a computer program product 200 (FIG. 7), and/or a method 300 (FIG. 8) may implement the gravity vector 28 and/or the coordinate diagram 30 to streamline robotic assisted surgeries. More particularly, the surgical system 10 may allow a medical professional, such as a surgeon "S," to gain up-to-date information on the current tilt about the longitudinal axis L(a) and the transverse axis T(a) of the operating table 16 based on graphical feedback from the display module 26. Further, as will be appreciated with further reading, the surgical system 10 may allow a surgeon S to modify the current tilt based on the graphical feedback without having to remove attention from the patient operating region 24 or the previous requirements of enlisting the help of another medical professional, such as a nurse or surgeon's assistant "N." In this manner, the gravity vector 28 only, the coordinate diagram 30 only, or both the gravity vector 28 and the coordinate diagram 30 may be implemented.

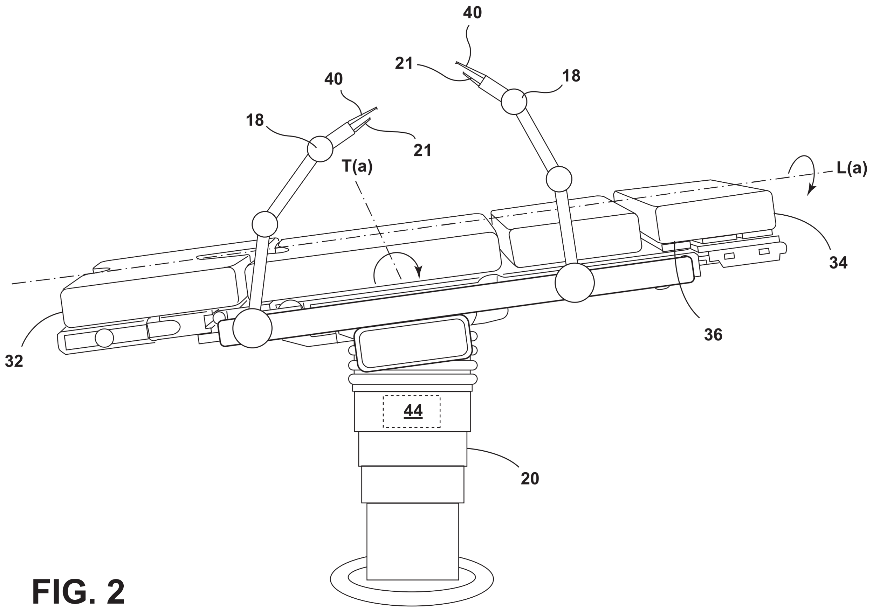

With reference now to FIGS. 1 and 2, the medical environment 12 may include an operating room where various robot-assisted medical procedures can be completed. Generally, the benefits associated with the surgical system 10, the control system 100, the computer program product 200, and/or the method 300 can be realized in any type of robot-assisted medical procedure where movement of the operating table 16 is utilized. More particularly, the operating table 16 may extend along the longitudinal axis L(a) between a first end 32 and a second end 34 and the transverse axis T(a) between a pair of sides 36. During usage, a patient "P" will generally lie on the operating table 16 lengthwise (e.g., foot to head) extending along the longitudinal axis L(a). The patient operating table 16 may include one or more straps 38 that secure the patient P to the operating table 16 as the operating table 16 is tilted about the longitudinal axis L(a) and the transverse axis T(a). Unless the patient operating region 24 (e.g., the portion of the patient's P body receiving the surgical intervention) is disposed directly under the pedestal 20 from which the patient operating table 16 articulates, the patient operating region 24 may move along a vertical axis V(a) as a result of tilting along the longitudinal axis L(a) and/or the transverse axis T(a). Further, portions of the operating table 16 will typically move along the vertical axis V(a) as the operating table 16 is tilted about the longitudinal axis L(a) and the transverse axis T(a), with the largest potential movements along the vertical axis V(a) at the first end 32, the second end 34, and/or the sides 36.

With continued reference to FIGS. 1 and 2, the at least one robotic arm 18 may include one, two, or more robotic arms 18 that each include various surgical tools 40. For example, these surgical tools may include a variety of cutting, lighting, suction, agitating, gripping, heating, cooling, lasering, and other functionalities. While the at least one robotic arm 18 is depicted as two robotic arms 18 located on a single side 36 of the operating table 16, it should be appreciated that the at least one robotic arm 18 may include one or more robotic arms 18 on one side 36 of the operating table 16 and one or more robotic arms 18 on a different side 36 of the operating table 16. In some implementations, the camera 21 is also located on the at least one robotic arm 18. For example, the camera 21 may be located proximate to the surgical tool 40 to provide up-to-date visual information on the procedure. In other implementations, one of the robotic arms 18 may include the camera 21 without a surgical tool 40. In some implementations, the camera 21 may be an endoscopic camera, fixed onto one of the robotic arms 18, and consisting of a fiber optical transmission inserted into the human body in order to capture pictures/video streams from inside the surgical spot or operating region 24. In such scenarios, the surgeon S is entirely focused on the image in the camera 21 and has no spatial reference. In this manner, the implementation of the graphical feedback (e.g., the gravity vector 28 and/or the coordinate diagram 30) described herein can be crucial to spatial understanding during an operation. In still further implementations, in addition to or alternatively from the camera 21 on the at least one robotic arm 18, the camera 21 may include other cameras located on a wearable device, such as a headpiece. Further, it should be appreciated that additional cameras may be in locations in addition to or alternatively from the at least one robotic arm 18 and/or wearable device. For example, a camera may be located on a moveable boom, statically connected within other locations in the medical environment 12, moveably connected to the operating table 16, a modular camera moveable around the medical environment 12 and positioned as needed, combinations thereof, and/or the like without departing from the scope of the subject disclosure. However, generally speaking, the graphical feedback (e.g., the gravity vector 28 and/or the coordinate diagram 30) may be particularly suitable for the endoscopic camera 21 located on the robotic arm 18 that moves with the operating table 16 along the vertical axis V(a), the longitudinal axis L(a), and the transverse axis T(a).

The operating table 16 may further include other tools, such as, the user interface 14 or a separate table interface 42. More particularly, the operating table 16 and robotic arms 18 may be a singular unit with the user interface 14 or separate units with separate interfaces 14, 42 in communication with the control system 100. The pedestal 20 may include one or more actuators 44 that tilt the operating table 16 along the longitudinal axis L(a) and/or the transverse axis T(a). For example, the actuator 44 may include movable pistons, gear driven arrangements, hydraulic systems, pneumatic systems, and/or the like for tilting the operating table 16 along the longitudinal axis L(a) and/or the transverse axis T(a). In some embodiments, tilting the operating table 16 is controlled by the user interface 14 and/or the table interface 42. For example, the user interface 14, the table interface 42, or the user interface 14 and the table interface 42 in combination may include a plurality of user inputs 46 for controlling the operation of the robotic arms 18, the cameras 21, the display module 26, the actuators 44, and other components of the surgical system 10 described herein. In the depicted arrangement, the plurality of user inputs 46 include a foot pedal that can be utilized for controlling the actuators 44 and tilting the operating table 16 along the longitudinal axis L(a) and/or the transverse axis T(a). Further, other inputs 46, for example, proximate the display module 26 may be utilized for controlling the robotic arms 18, the cameras 21, and specifics of the image data 22 generated on the display module 26. In this manner, the surgeon S may be able to simultaneously work the foot pedal while viewing the display module 26 and utilizing the other inputs to control the robotic arms 18 during a surgical procedure. Further it should be appreciated that the operating table 16 may be tilted along other axes (e.g., diagonal axes) than those depicted and/or a combination of the longitudinal axis L(a) and/or the transverse axis T(a) without departing from the present disclosure. However, generally, rotation along both the longitudinal axis L(a) and the transverse axis T(a) may be used to obtain different positions such as diagonal tilting.

In some implementations, the current position of the operating table 16 may be determined by the image data 22, information from the pedestal 20, actuators 44, and/or one or more positional sensors 45. For example, the one or more positional sensors 45 may be located in the pedestal 20 (e.g., the actuators 44), the operating table 16, and/or combinations thereof. In some implementations, the one or more sensors 45 may further or alternatively be remote sensors 45 capable of determining the position of the operating table 16 via 2D or 3D vision systems, such as stereovision, time-of-flight, and/or the like.

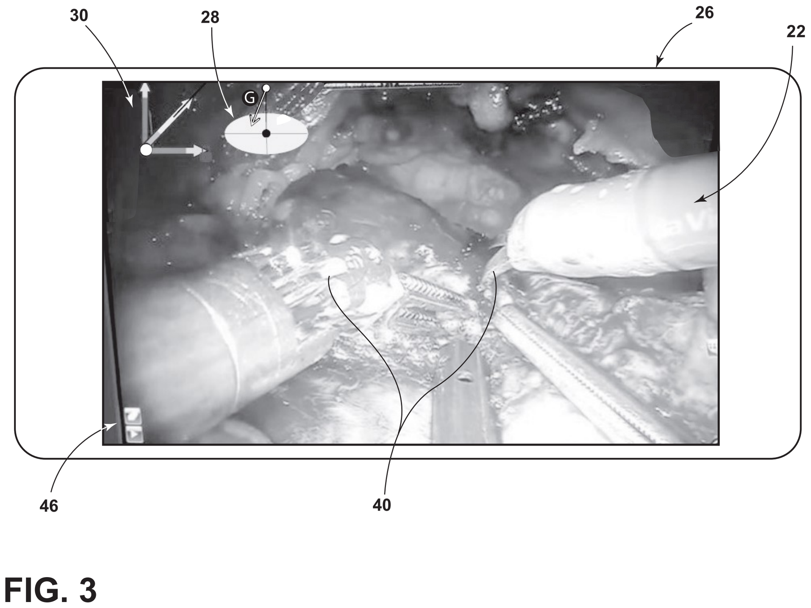

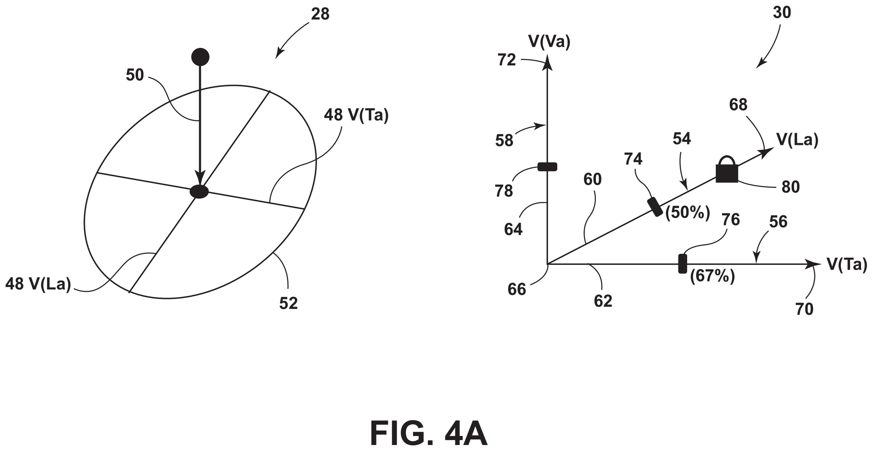

With reference now to FIGS. 3-4C, the display module 26 is depicted with the image data 22 (e.g., real time or near real time images captured by the camera 21) generated thereon and with the gravity vector 28 and the coordinate diagram 30 overlaid over the image data 22. Various usage scenarios will be described in relation to FIGS. 4A-4C. In the depicted implementation, the gravity vector 28 includes a diagram 48 of a virtual representation of controller movement (e.g., degrees of freedom) in a first controller axis V(La) and a virtual representation of controller movement (e.g., degrees of freedom) in a second controller axis V(Ta). As will be appreciated with further reading, input to the controller, such as the user interface 14 (e.g., the joystick), in the first and second controller axes V(La) and V(Ta) may be translated to real-world movement of the robotic arm 18, operating table 16, and/or surgical tool 40. Likewise, the first and second controller axes V(La) and V(Ta) may be generated on the display module 26 (and interfaced with) as a guide for the surgeon S throughout the operation. The gravity vector 28 may further include a marking 50 positioned relative to the diagram 48 of the virtual representation of controller movement in the first controller axis V(La) and the virtual representation of controller movement in the second controller axis V(Ta) that corresponds to the current tilt with respect to gravity. For example, the marking 50 may include an arrow (e.g., a vector) that a vector exhibits both direction and magnitude of the current tilt.

In the depicted arrangement, the gravity vector 28 includes an outer boundary 52 corresponding to an allowable amount of tilt. The outer boundary 52 may generally have an elliptical shape. More particularly, the magnitude that the operating table 16 is tilted along the longitudinal axis L(a) may limit the amount of tilt available along the transverse axis T(a), where a greater magnitude of tilting along the longitudinal axis L(a) may correspond to less available tilting along the transverse axis T(a). Likewise, the magnitude that the operating table 16 is tilted along the transverse axis T(a) may limit the amount of tilt available along the longitudinal axis L(a), where a greater magnitude of tilting along the transverse axis T(a) may correspond to less available tilting along the longitudinal axis L(a). Further, depending on an entry point and the angle of the camera 21 (e.g., an endoscopic camera located on the robotic arm 18), the camera 21 may not always be positioned in line with the physical axes T(a), L(a). For example, if the camera 21 is facing the longitudinal axis L(a) inputs on the user interface 14 (e.g., joystick) may be inverted or otherwise shifted as compared to a situation where the camera 21 is facing the transverse axis T(a) or any intermediate positions and angles. In this manner, the diagram 48 of the virtual representation of controller movement in the first controller axis V(La) and the virtual representation of controller movement in the second controller axis V(Ta) may represent a guide for the surgeon S during the procedure to translate movements of the robotic arm 18, operating table 16, and/or surgical tool 40 via the user interface 14 to the real-world patient operating region 24. The virtual representation of controller movement in the first controller axis V(La) and the virtual representation of controller movement in the second controller axis V(Ta) may intersect (e.g., centrally) within the outer boundary 52 and form quadrants. However, it should be appreciated that the outer boundary 52 may include shapes other than elliptical. For example, the outer boundary 52 may be square, circular, and/or other shapes without departing from the scope of the subject disclosure. It should also be appreciated that the marking 50 may have shapes other than the arrow. For example, the marking 50 may be a shape (e.g., circle, square, or other shape) that floats within the outer boundary 52, where the closer the marking 50 is to the boundary 52, the greater magnitude of tilt of the operating table 16 along the longitudinal axis L(a) and the transverse axis T(a).

With continued reference to FIGS. 3-4C, the user interface 14 (e.g., the user inputs 46) may be, at least in part, generated on the display module 26, e.g., via touchscreen inputs. In some embodiments, the gravity vector 28 can be interfaced with (e.g., via touchscreen inputs or other user inputs 46) to modify the current tilt of the operating table 16. In some implementations, the gravity vector 28 can be interfaced with and rotated to align the virtual axes V(Ta) and V(La) with the real-world axes L(a) and T(a). In some implementations, the gravity vector 28 can be interfaced with and moved to different positions in the display module 26, resized (e.g., enlarged or downsized). For example, a surgeon S or other caregiver (e.g., nurse N) may select a portion within the outer boundary 52 of a desired tilt along the longitudinal axis L(a) and/or the transverse axis T(a). In response, the control system 100 translates the input into the real-world axes L(a), T(a) and may automatically control the actuators 44 to move the operating table 16 or another input may be required. For example, once the desired tilt is selected, the surgeon S may interface with a different user input 46 (e.g., the foot pedal or joystick) to slowly move the operating table 16 in the desired direction, where movements along the virtual axes V(Ta), V(La) are translated to the real-world axes L(a), T(a). In this manner, the movement of the operating table 16 can be terminated once a desired tilt is obtained (e.g., based on the visibility of the patient operating region 24). In some embodiments, the touchscreen inputs on the display module 26 may include a slider or other input that permits the surgeon to change the tilt of the operating table 16 without directly interfacing with the gravity vector 28 and solely utilizing the gravity vector 28 as a relative positional guide between the camera 21 and the operating table 16. In still further embodiments, the surgeon S may utilize any user inputs 44 on the user interface 14 and/or table interface 42, such as a joystick, buttons, a mouse, or other inputs to tilt the operating table 16. Regardless of how the operating table 16 is tilted, the gravity vector 28 can provide real time or near real time positional data. The gravity vector 28 may be selectively generated and removed from the image data 22.

With reference now to FIGS. 3-4C, the coordinate diagram 30 is depicted. More particularly, the control system 100 may be configured to graphically generate the coordinate diagram 30 over the image data 22 displayed on the display module 26. The coordinate diagram 30 may correspond to an allowable remaining tilt of the operating table 16 along the real-world axes L(a), T(a) and/or the degrees of freedom available along the virtual axes V(Ta), V(La). For example, the coordinate diagram 30 may include a first axis diagram 54 corresponding to the virtual longitudinal axis V(La) and a second axis diagram 56 corresponding to the virtual transverse axis V(Ta). Further, in some embodiments, the coordinate diagram 30 may further include a third axis diagram 58 corresponding to controller movement (e.g., degrees of freedom) along a vertical representation of controller movement in a third controller axis V(Va). The first axis diagram 54 may include a first diagram marker 60 associated with the current tilt along the first vertical axis V(La) and the second axis diagram 56 may include a second diagram marker 62 associated with the current tilt along the second virtual axis V(Ta) transverse axis T(a). Likewise, when the third axis diagram 58 is utilized, the third axis diagram 58 may include a third diagram marker 64 associated with the current position along the third virtual axis V(Va). More particularly, the axis diagrams 54, 56, 58 may represent a guide for the surgeon S during the procedure to translate movements (e.g., visually by the surgeon S or automatically via the control system 100) of the robotic arm 18, operating table 16, or the surgical tool 40 via the user interface 14 to the real-world patient operating region 24.

The first diagram marker 60 may include a first colorization of a segment along the first axis diagram 54, where the length of the segment corresponds to the remaining available movement of the controller (e.g., user interface 14) along the first virtual axis V(La). The second diagram marker 62 may include a second colorization of a segment along the second axis diagram 56, where the length of the segment corresponds to the remaining available tilt movement of the controller (e.g., user interface 14) along the second virtual axis V(La). The third diagram marker 64 may include a third colorization of a segment along the second axis diagram 56, where the length of the segment corresponds to the remaining available movement of the controller (e.g., user interface 14) along the third virtual axis V(a). As explained previously, movement along the vertical axis V(a) may be dependent on the location of the patient operating region 24 relative to the pedestal 20. The first colorization, the second colorization, and the third colorization may be different in color. More particularly, the first axis diagram 54, the second axis diagram 56, and the third axis diagram 58 may be generated as lines or arrows, which may extend outwardly from a common point 66. In some implementations, the first axis diagram 54 is generated in a first color, the second axis diagram 56 is generated in a second color, and the third axis diagram 58 is generated in a third color. The first, second, and third colors may be different. In some embodiments, the first colorization corresponds to highlighting (e.g., brightening) or darkening the first color in the segment of the first axis diagram 54. In some embodiments, the second colorization corresponds to highlighting or darkening the second color in the segment of the second axis diagram 56. In some embodiments, the third colorization corresponds to highlighting or darkening the third color in the segment of the third axis diagram 58.

With continued reference to FIGS. 3-4C, the first axis diagram 54 may include a first diagram limit 68 indicating the maximum tilt along the first virtual axis V(La) and the second axis diagram 56 may include a second diagram limit 70 indicating the maximum tilt along the second virtual axis V(Ta). Likewise, when the third axis diagram 58 is utilized, the third axis diagram 58 may include a third diagram limit 72 indicating the maximum movement along the third virtual axis V(Va). The first diagram limit 68, the second diagram limit 70, and the third diagram limit 72 may correspond to terminal ends of the lines or arrows. In this manner, the surgeon S can quickly obtain a visualization of the remaining available tilt or movement of the robotic arm 18, the surgical tool 40, and/or the operating table 16 in the real-world axes L(a), T(a) from the display module 26.

Similar to the gravity vector 28, in some embodiments, the coordinate diagram 30 can be interfaced with (e.g., via touchscreen inputs or other user inputs 46) to modify the current tilt of the operating table 16 (e.g., via translation between the virtual and real-world axes). For example, a surgeon or other caregiver (e.g., nurse N) may select a portion of the first axis diagram 54, the second axis diagram 56, and the third axis diagram 58. More particularly, the user interface 14 may be configured to select the coordinate diagram 30 and, via inputs to the user interface 14 (e.g., via touchscreen inputs or other user inputs 46), modify the current tilt of the operating table 16. For example, in some implementations, the first axis diagram 54 may include a first modification marker 74 that is interactable and indicates a desired tilt or movement along the first virtual axis V(La) and the second axis diagram 56 may include a second modification marker 76 that is interactable and indicates a desired tilt or movement along the second virtual axis T(a). In implementations with the third axis diagram 58, the third axis diagram 58 may include a third modification marker 78 that is interactable and indicates a desired movement along the third virtual axis V(Va). In this manner, the first modification marker 74 may be moveable (e.g., slidable) along the first axis diagram 54, the second modification marker 76 may moveable (e.g., slidable) along the second axis diagram 56, and the third modification marker 78 may be moveable (e.g., slidable) along the third axis diagram 58.

The coordinate diagram 30 may also include a lock 80 that can be utilized by inputs directly to the coordinate diagram 30 (e.g., via the touchscreen) or indirectly (via other user inputs 44). The lock 80 may be applied to the first axis diagram 54, the second axis diagram 56, and/or the third axis diagram 58 and ultimately selectively modifies the first diagram maximum 68, the second diagram maximum 70, and/or the third diagram maximum 72. Such functionalities may be beneficial, for example, if the surgeon S finds movement to tilt along a particular axis more than ideal for visibility of the patient operating region 24, then the lock 80 can be set to a more ideal position to prevent over tilting or movements via the user interface 14 during the subsequent parts of the operation. In this manner, the caregiver N and/or surgeon S has the option to lock the tilt or movement of the operating table 16 along one or more of the virtual axes and only tilt or move the operating table 16 along whichever axis or axes are not locked by the lock 80.

In response to inputs directly to the coordinate diagram 30 (e.g., via the touchscreen) or indirectly (via other user inputs 46), the control system 100 may automatically control the actuators 44 to move the operating table 16 or another input may be required. For example, once the desired tilt is selected, the surgeon S may interface with a different user input 46 (e.g., the foot pedal) to slowly move the operating table 16 in the desired direction. In this manner, the movement of the operating table 16 can be terminated once a desired tilt is obtained (e.g., based on visibility of the patient operating region 24). In some embodiments, the touchscreen inputs on the display module 26 may include a slider or other input that permits the surgeon to change the tilt of the operating table 16 without directly interfacing with the coordinate diagram 30. In still further embodiments, the surgeon S may utilize any user inputs 46 on the user interface 14 and/or table interface 42, such as a joystick, buttons, a mouse, or other inputs to tilt the operating table 16. Regardless of how the operating table 16 is tilted, the coordinate diagram 30 can provide real time or near real time positional data. In some implementations, the coordinate diagram 30 can be interfaced with and rotated to align the virtual axes V(Ta), V(La) with the real-world axes L(a), T(a). In some implementations, the coordinate diagram 30 can be interfaced with and moved to different positions in the display module 26, resized (e.g., enlarged or downsized). The coordinate diagram 30 may be selectively generated and removed from the image data 22.

With reference now specifically to FIGS. 4A-4C, various example use scenarios are illustrated. In FIG. 4A, the patient operating table 16 is level (e.g., no articulation about axes T(a), L(a)) and the camera 21 is inserted into the patient operating region 24 from a direction of the patient's head and is aligned with the longitudinal axis L(a). In this manner, the gravity vector 28 marking 50 is perpendicular to the orientation of the camera 21 (e.g., because the camera 21 orientation is perpendicular to real-world gravity forces) and extends to the intersection between the virtual axes V(La), V(Ta). Based on this orientation of the camera 21, forward movement from the user interface 14 (e.g., pushing a joystick forward) will result in tilting the operating table 16 along the longitudinal axis L(a) (e.g., articulating along the transverse axis T(a)) and, more particularly, raising the patient's feet and lowering the patient's head. As expected, backward movement from the user interface 14 (e.g., pulling a joystick back) will result in opposite movement of the operating table 16. Further, sideways movement from the user interface 14 (e.g., pushing a joystick left or right) will result in similar movement along the along the transverse axis T(a) via articulating the operating table 16 along the longitudinal axis L(a). In the leveled position, the patient operating table 16 has 50% of articulation available about opposing directions of the longitudinal axis L(a) and opposing directions about the transverse axis T(a).

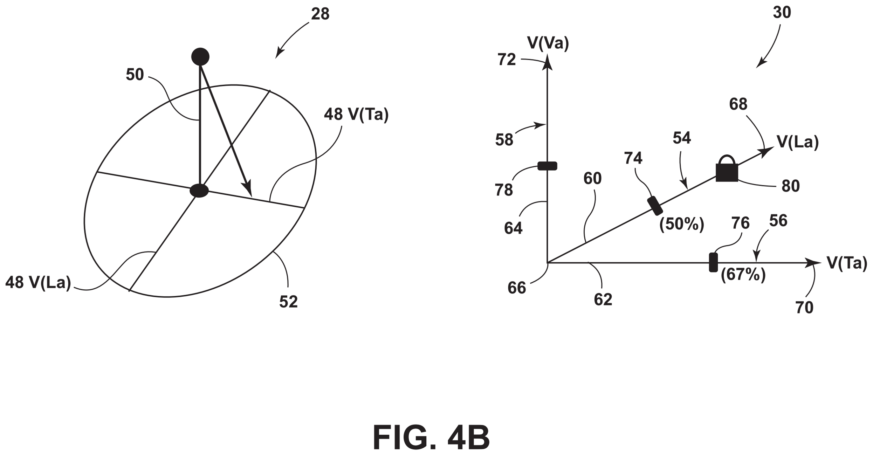

With reference now to FIG. 4B, assume a scenario where the operating table 16 and camera 21 are in the leveled position represented in FIG. 4A, and now during the procedure the surgeon S desires to move organs away from the surgical field ("deeper into the picture"). In this manner, intuitively, the surgeon S can utilize the user interface 14 (e.g., push the joystick forward, without requiring knowledge of the physical orientation of the operating table 16, camera 21, etc. In FIG. 4B, the operating table 16 has been articulated at +15° along the longitudinal axis L(a). In response, these movements of relative positions are visualized via changes to the gravity vector 28 and the coordinate diagram 30. More particularly, the surgeon S can see (e.g., via the coordinate diagram 30) that the recent movement consumed about 17% stroke and 33% in articulation in the same direction are still available. More particularly, assuming 90° of total movement are available (e.g., 45° in each opposite direction), a 15° movement still permits an additional 30° of articulation before articulation in that direction is maxed out. The additional 30° of articulation can be translated to a percentage of the allowable remaining tilt or (30°/90° = 33%). At the same time, the marker 50 of the gravity vector 28 is moved along the virtual longitudinal axis V(Ta) so that the surgeon S can quickly gain a frame of reference and objects in the FOV of the camera 21 will tend to move deeper into the picture of the display module 26 or generally away from the surgeon S and/or the FOV.

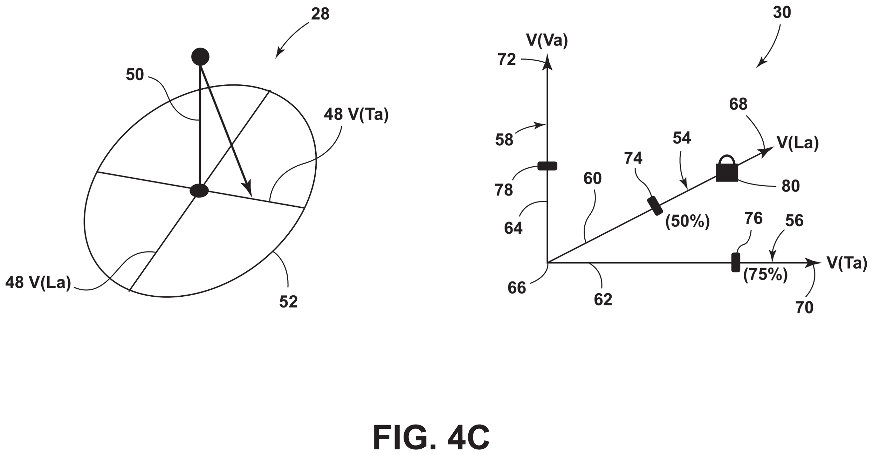

With reference now to FIG. 4C, assume a scenario where the operating table 16 and camera 21 are in the leveled position represented in FIG. 4A but the camera 21 is inserted into a side of the patient operating region 24 and is oriented along the transverse axis T(a). The control system 100 may be configured to align the virtual longitudinal axis V(La) with real-world transverse axis T(a) and align virtual transverse axis V(Ta) with real-world longitudinal axis L(a). In this manner, forward and backward inputs of the user interface 14 (e.g., bending the joystick forward/backward) translate into movement along the transverse axis T(a), thus permitting the patient to move or roll to their right or left side accordingly. Similarly, right or left inputs of the user interface 14 (e.g., bending the joystick left or right) will lower patient's feet and raise his head or vice versa. These movements during surgery are translated by the control system 100 to deliver real-time feedback on the display module 26. In FIG. 4C, movement of the operating table 16 is shown as +15° along the transverse axis T(a). Assuming only 60° of total movement is available (e.g., 30° in each opposite direction), a 15° movement still permits an additional 15° of articulation before articulation in that direction is maxed out. The additional 15° of articulation can be translated to a percentage of the allowable remaining tilt or (15°/60° = 25%), when considering the changed translation parameters.

In the depicted scenarios in FIGS. 4A-4C, the surgeon S does not need to divert attention to the real-life operating table 16 position but instead focus on the virtual graphics on the display module 26. It should be appreciated that these scenarios are simplified in terms of the alignment of the camera 21. However, the same principles apply to scenarios where the camera 21 enters the patient operating region 24 at random angles, which may be adjusted and interacted with the surgeon S over the course of the procedure. Because the camera 21 is held by the robotic arm 18, the control system 100 can adjust the translation between real-world axes L(a), T(a) and the virtual axes V(La), V(Ta) accordingly.

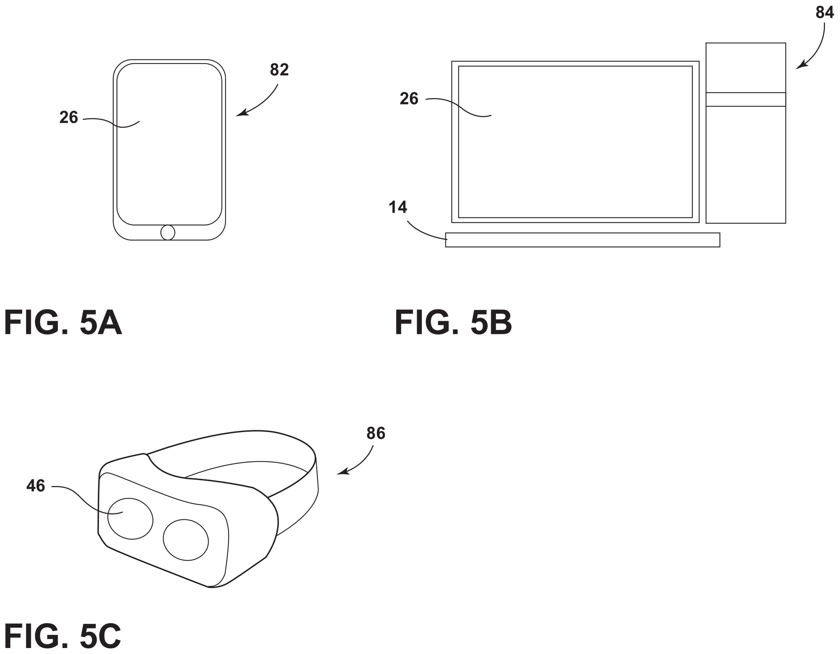

With reference now to FIGS. 5A-5C, the display module 26 may be implemented in a variety of technologies. For example, in FIG. 5A, the display module 26 may be implemented in a modular device 82, such as a tablet, laptop, or other modular electronic device, that can be selectively moved around the medical environment. In some implementations, the patient operating table 16 or other components of and in the medical environment 12 may include connection brackets or holders for selectively mounting modular devices 82. In other implementations, such as that depicted in FIG. 5B, the display module 26 may be implemented in a non-modular device 84, such as a computer, a permanently mounted electronic device, or other non-modular electronic devices that is/are located around the medical environment 12. In still further implementations, the display module 26 may be implemented as a wearable visualization device 86, such as a semi-virtual or augmented reality device, as depicted in FIG. 5C. In some embodiments, the camera 21 may be located on the visual visualization device 86. While FIGS. 5A-5C provides examples of technologies that can be utilized with the display module 26, it should be appreciated that the display module 26 may be implemented in a variety of devices other than those depicted in FIGS. 5A-5C and, in particular, displays utilized in endoscopic surgeries. Each of these example technologies may include at least some of the variety of user inputs 46.

FIG. 6 illustrates the control system 100 associated with, for example, the surgical system 10 (e.g., the patient operating table 16, the robotic arms 18, the pedestal 20, the camera 21, the display module 26, the surgical tool 40, the actuators 44, the one or more sensors 45, and other components of the surgical system 10 and/or the medical environment 12). As will be appreciated with further reading, the control system 100 may also be associated with and/or receive instructions from a computer program product 200 that includes instructions to carry out the methods and functionalities described herein. The control system 100 may include an electronic control unit (ECU) 102. The ECU 102 may include the processor 104 and a memory 106. The processor 104 may include any suitable processor 104. Additionally, or alternatively, the ECU 102 may include any suitable number of processors, in addition to or other than the processor 104. The memory 106 may comprise a single disk or a plurality of disks (e.g., hard drives), and includes a storage management module that manages one or more partitions within the memory 106. In some embodiments, the memory 106 may include flash memory, semiconductor (solid state) memory, a database accessible via a cloud, a non-transitory storage medium, a combination thereof, and/or the like. The memory 106 may include Random Access Memory (RAM), a Read-Only Memory (ROM), or a combination thereof. The memory 106 may include at least some instructions received from the computer program product 200.

The memory 106 may include instructions that, when executed by the processor 104, cause the processor 104 to, at least, perform the functions and method steps as described herein. For example, the control system 100 may be configured to generate the gravity vector 28 and/or coordinate diagram 30 on the display module 26 (e.g., from the one or more sensors 45) and receive instructions to interface with the gravity vector 28 and/or coordinate diagram 30, receive instructions from the user interface 14 and/or the table interface 42, selectively remove the gravity vector 28 and/or coordinate diagram 30, and generally receive real time or near real time positional data (e.g., via the one or more sensors 45). In some embodiments, the control system 100 is a global control system in communication with a plurality of ECUs 102 (e.g., associated with the user interface 14, the patient operating table 16, the robotic arms 18, the pedestal 20, the camera 21, the display module 26, and/or devices associated therewith, the surgical tool 40, the table interface 42, the actuators 44, the one or more sensors 45, and/or other electronic components of the surgical system 10. In some embodiments, the control system 100 is located in in the operating table 16, devices (e.g., FIGS. 5A-5C) associated with the display module 26, user interface 14, the table interface 42, and/or the like and receives information from the surgical system 10 (e.g., the one or more sensors 45, the user interface 14, the table interface 42) and the computer program product 200.

With continued reference to FIG. 6, the memory 106 may contain software 108, a medical environment module 110, a medical device module 112, a personnel module 114, a protocol dictionary 116, a display graphics module 118, and a communications module 120. More particularly, the software 108 may have updatable instructions that operate various features of the surgical system 10 in a remote or locally based application. The software 108 may include instructions for generating global statistics about the system 10 or local statistics related to the medical environment 12, a branch of the medical environment, medical devices within the medical environment 12, updates on regulation information, and updates to the control system 100 operational protocols.

The medical device module 112 may include information about and instructions related to interfacing with the user interface 14 (e.g., the device and associated user inputs 46), the patient operating table 16 (e.g., type, dimensions, etc.), the robotic arms 18 (e.g., instructions for moving the robotic arms 18), the pedestal 20 (e.g., instructions related to moving and/or tiling the operating table 16), the camera 21 (e.g., instructions for capturing the image data 22), the display module 26 and/or devices associated therewith, the surgical tool 40, the table interface 42, the actuators 44, the one or more sensors 45, and/or other electronic components of the surgical system 10.

The personnel module 114 may include information about surgeon S or caregiver N interfacing with the surgical system 10. For example, the personnel module 114 may include limitations for controlling certain components of the surgical system 10 based on the assigned responsibilities of the assigned surgeon S or caregiver N. For example, certain responsibilities may be assigned to different personnel in the medical environment 12. The personnel module 114 may further include and/or log name, employment position, responsibilities, shift details, movement paths, communications, and interactions with medical devices. The personnel module 114 may also include different profiles for different surgeon S or caregiver N preferences. For example, a surgeon S may prefer to only utilize one of the gravity vector 28 or the coordinate diagram 30. In such instances, the control system 100 may receive a request for a profile (e.g., via user inputs 46) or automatically assign the profile based on the assigned surgeon S or caregiver N. More particularly, the control system 100 may be configured to automatically generate whichever of the gravity vector 28 or the coordinate diagram 30 that the surgeon S or caregiver N prefers and remove either the gravity vector 28 or the coordinate diagram 30 that the surgeon S or caregiver N does not typically utilize (e.g., which can be re-generated upon further user inputs 46). In other examples, the surgeon S or caregiver N may have preferences related to the specifics of the gravity vector 28 or the coordinate diagram 30, for example, the shape, size, and color of the gravity vector 28 and coordinate diagram 30 that is overlaid on the image data 22.

The protocol dictionary 116 may include information related to the specific medical operation or a particular patient P need. For example, certain types of medical operations or health status of the patient P may require or be optimal by limiting the amount of movement and/or tilt of the operating table 16. In this manner, the protocol dictionary 116 may set limitations to movement of the operating table 16, which may be visually depicted in the outer boundary 52 of the diagram vector 28 and/or the first diagram limit 68, the second diagram limit 70, and the third diagram limit 72 of the coordinate diagram 30.

The display graphics module 118 may include information and instructions related to overlaying the gravity vector 28 and coordinate diagram 30 on the image data 22 (e.g., if they are generated over the image data 22). The display graphics module 118 may further include instructions regarding the shape, size, orientation, and color of the gravity vector 28 and coordinate diagram 30. For example, the colors of the first colorization, the second colorization, and the third colorization may be modified. In other examples, any touchscreen inputs, such as the first modification marker 74, the second modification marker 76, the third modification marker 78, and the lock 80 may be modified based on different and available interfacing options (e.g., a rotational wheel, tilting based on inputting numbers, selectable arrows, etc.). In this manner, the display graphics module 118 may include a list of all available modifications of the gravity vector 28 and coordinate diagram 30, that may be regularly updated and may be saved in the profiles in the personnel module 114. The display graphics module 118 may further include translational algorithms to translate controller movements along the virtual axes V(La), V(Ta) into real-world axes L(a), T(a) or vise-versa, and provide real-time or near real-time information on the relative positioning of the camera 21 with respect to the operating region 24.

The communications module 120 may include instructions for communications between the control system 100 and components of the surgical system 10. For example, the communications module 120 may communicate via wired or wireless information to the user interface 14, the patient operating table 16, the robotic arms 18, the pedestal 20, the camera 21, the display module 26, and/or devices associated therewith, the surgical tool 40, the table interface 42, the actuators 44, the one or more sensors 45, and/or other electronic components of the surgical system 10, and/or associated ECUs 102 of the components of the surgical system 10.

With reference now to FIG. 7, in one example, the aforementioned computer program product 200 may include many of the instructions associated with the memory 106. More particularly, the instructions included in the computer program product 200 may include instructions (e.g., saved in non-transitory memory) related to creating and generating the gravity vector 28 and/or coordinate diagram 30, including functions associated therewith, and linking information. More particularly, the computer program product 200 may link information between the user interface 14, the patient operating table 16, the robotic arms 18, the pedestal 20, the camera 21, the display module 26, and/or devices associated therewith, the surgical tool 40, the table interface 42, the actuators 44, the one or more sensors 45, and/or other electronic components of the surgical system 10, and/or associated ECUs 102 of the components of the surgical system 10. For example, the computer program product 200 may include instructions to perform the method steps and functions described herein, but may rely on certain features/inputs such as from the one or more user interface 14, the patient operating table 16, the robotic arms 18, the pedestal 20, the camera 21, the display module 26, and/or devices associated therewith, the surgical tool 40, the table interface 42, the actuators 44, the one or more sensors 45, and/or other electronic components of the surgical system 10, and/or associated ECUs 102 of the components of the surgical system 10

Further, select or all features of the software 108, the medical environment module 110, the medical device module 112, the personnel module 114, the protocol dictionary 116, and the communications module 120 may be contained on the control system 100 while the computer program product 200 includes the display graphics module 118. However, in some embodiments, the computer program product 200 may include instructions for the surgical system 10 to collect and store certain features/inputs such as from the user interface 14, the patient operating table 16, the robotic arms 18, the pedestal 20, the camera 21, the display module 26, and/or devices associated therewith, the surgical tool 40, the table interface 42, the actuators 44, the one or more sensors 45, and/or other electronic components of the surgical system 10, and/or associated ECUs 102 of the components of the surgical system 10 and information collected and contained in memory 106.

The computer program product 200 may include, for instance, one or more computer-readable medium 202 (e.g., non-transitory memory) to store computer-readable program code means or logic 204 in order to provide and facilitate one or more functions and method steps described in the present disclosure. The program code contained or stored in/on a computer-readable medium 202 can be obtained and executed by a computer, such as the control system 100 to behave/function in a particular manner. The program code can be transmitted using any appropriate medium, including (but not limited to) wireless, wireline, optical fiber, and/or radiofrequency. The program code 204 includes instructions for carrying out operations to perform, achieve, or facilitate aspects of the disclosure may be written in one or more programming languages. In some embodiments, the programming language(s) include object-oriented and/or procedural programming languages such as C, C++, C#, Java, and/or the like. The program code 204 may execute entirely on the user's device (e.g., devices depicted in FIGS. 5A-5C, the user interface 14, the table interface 42, or other devices in the surgical system 10), entirely remote from the user's computer, or a combination of partly on the user's computer and partly on a remote computer. In some embodiments, a user's computer and a remote computer are in communication via a network such as a local area network (LAN) or a wide area network (WAN), and/or via an external computer (for example, through the Internet using an Internet Service Provider).

In one example, the program code 204 includes one or more program instructions obtained for execution by one or more processors (e.g., the processor 104). The instructions contained in the program code 204 may be provided to the one or more processors of, for example, one or more computer systems (e.g., devices depicted in FIGS. 5A-5C, the user interface 14, the table interface 42, or other devices in the surgical system 10), such that the program instructions, when executed by the one or more processors, perform, achieve, or facilitate aspects of the functionalities and methods described herein. The descriptions, figures, and flowcharts, and claims depicted and described herein illustrate the architecture, functionality, and operation of possible embodiments of surgical system 10, methods, and/or instructions contained on the computer program product 200 or memory 106 according to various aspects of the present disclosure.

In some embodiments, as noted above, descriptions, actions, functions, figures, and flowcharts depicted and described herein may represent and/or be the result of a module, segment, or portion of code, which includes one or more executable instructions for implementing the specified behaviors and/or logical functions. It should be appreciated that actions, functions, and blocks described herein may occur in a different order than depicted and/or described, or may occur simultaneous to, or partially/wholly concurrent with, one or more actions, functions, and blocks. For example, two actions or method steps (e.g., blocks) shown and/or described in succession may, in fact, be executed substantially concurrently or in the reverse order.

FIG. 8 illustrates a method 300 of visually guiding (e.g., via the display module 26) a surgeon S in tilting an operating table 16 about a longitudinal axis L(a) and a transverse axis T(a). The method 300 may be carried out utilizing the surgical system 10, the control system 100, and/or the computer program product 200. At step 302, the method 300 includes receiving, from the operating table 16, a current tilt of the operating table 16 about the longitudinal axis L(a) and the transverse axis T(a). Step 302 may include generating image data from a camera 21 on a display module 26. The image data 22 may correspond to a patient operating region 24 of a patient P in surgery.

At step 304, the method 300 includes graphically generating at least one of a gravity vector 28 or a coordinate diagram 30. More particularly, the gravity vector 28 may correspond to the current tilt on the display module 26 and the coordinate diagram 30 may correspond to the allowable remaining tilt. Step 304 may include overlaying the at least one of the gravity vector 28 or the coordinate diagram 30 over the image data 22 generated on the display module 26.

Step 304 may further include, in regards to generating the gravity vector 28, generating a diagram 48 of the virtual representation of controller movement in the first controller axis V(La) and the virtual representation of controller movement in the second controller axis V(Ta) and a marking 50 positioned relative to the diagram 48 of the virtual representation of controller movement in the first controller axis V(La) and the virtual representation of controller movement in the second controller axis V(Ta) associated with the current tilt and relative forces of gravity with respect to the FOV of camera 21. The marking 50 may be a vector exhibiting both direction and magnitude of the current tilt. In some embodiments, the method 300 may include generating an outer boundary 52 corresponding to an allowable amount of tilt, and the diagram 48 of the virtual representation of controller movement in the first controller axis V(La) and the virtual representation of controller movement in the second controller axis V(Ta) may intersect within the outer boundary 52. The outer boundary 52 may be generated with an elliptical shape.

Step 304 may further include, with regard to generating the coordinate diagram 30, showing a current tilt. More particularly, the coordinate diagram 30 may include a first axis diagram 54 of a first color corresponding to the first virtual axis V(La) and a second axis diagram 56 of a second color that is different than the first color corresponding to the second virtual axis V(Ta). In some embodiments, a third axis diagram 58 is generated as previously described. The position of the camera 21 may be translated and visualized as a first diagram marker 60 that includes, for example, a first colorization of a segment along the first axis diagram 54, where the length of the segment is associated with the current tilt along the longitudinal axis L(a) and/or degrees of freedom of the first virtual axis V(La). The first colorization may include a brightened first color. Similarly, the current tilt along the transverse axis T(a) may be visualized as a second diagram marker 62 that includes a second colorization of a segment along the second axis diagram 56, where the length of the segment is associated with the current tilt along the transverse axis T(a) and/or degrees of freedom of the second virtual axis V(Ta). The second colorization may include a brightened second color. In some embodiments, the first axis diagram 54 may include a first diagram limit 68 indicating the allowable remaining movement along the first virtual axis V(La) and the second axis diagram 56 may include a second diagram limit 70 indicating the allowable remaining movement along the second virtual axis V(Ta). More particularly, the first diagram limit 68 may include a terminal end of the first axis diagram 54 and the second diagram 48 limit may include a terminal end of the second axis diagram 56, which may be presented as lines or arrows. The third axis diagram 58 may have similar features as previously described.

At step 306, the method 300 may include receiving inputs from a user, such as a surgeon S, from one or more user inputs to modify the tilt of the operating table 16, move the robotic arm 18, and/or otherwise change relative positioning between the operating region 24 and the camera 21. For example, step 306 may include receiving an input from a user input 46 located on a user interface 14 (e.g., the joystick), a table interface 42, and/or touch inputs from the display module 26. In some embodiments, the user input 46 may be received by directly interfacing with the gravity vector 28 and/or the coordinate diagram 30 (e.g., on a touchscreen interface) as described above. In some embodiments, the user input 46 may be received by indirectly interfacing with the gravity vector 28 and/or the coordinate diagram 30 via one or more user inputs 46 (e.g., the joystick) spaced from the gravity vector 28 and/or the coordinate diagram 30 as described above.

At step 308, the method 300 may include translating inputs with respect to the virtual axes V(La), V(Ta) with respect to the real-world axes L(a), T(a) and providing guiding feedback on one or both of the gravity vector 28 and/or the coordinate diagram 30. More particularly, step 308 may include generating a lock selectively implementable on the first axis diagram and/or second axis diagram. Further, in some embodiments, the lock may be moveable along the first axis diagram and second axis diagram proportional to the limit of tilt.

Embodiments of the invention can be described with reference to the following numbered clauses, with preferred features laid out in the dependent clauses:
1. A surgical system comprising:
   a user interface;
   an operating table supported on a pedestal that can selectively tilt, based on instructions from the user interface, the operating table about a longitudinal axis and a transverse axis;
   at least one robotic arm supported by the operating table and moveable relative to the operating table via the user interface;
   a camera connected to the robotic arm and configured to capture image data within a field-of-view ("FOV") of a patient operating region;
   a display module configured to display the image data; and
   a control system configured to:
      receive a current tilt of the operating table about the longitudinal axis and the transverse axis; and
      graphically generate a gravity vector over the image data displayed on the display module, the gravity vector including one or more virtual axes corresponding to degrees of freedom available on the user interface with respect to the FOV.
2. The surgical system of clause 1, wherein the one or more virtual axes include a diagram of a virtual representation of controller movement in the first virtual axis and a virtual representation of controller movement in the second virtual axis.
3. The surgical system of clause 2, wherein the gravity vector further includes a marking positioned relative to the first and second virtual axes that corresponds to the current forces of gravity with respect to the FOV.
4. The surgical system of clause 3, wherein the marking includes an arrow.
5. The surgical system of clause 4, wherein the arrow is a vector exhibiting both direction and magnitude of the current tilt.
6. The surgical system as in one of clauses 1-5, wherein the gravity vector includes an outer boundary corresponding to an allowable amount of tilt.
7. The surgical system as in one of clauses 1-5, wherein the user interface is, at least in part, generated on the display module and the gravity vector can be interfaced with to modify the current tilt of the operating table.
8. The surgical system as in one of clauses 1-5, wherein the gravity vector is selectively generated and removed from the image data.
9. The surgical system of clause 1, wherein the control system is further configured to:
   graphically generate a coordinate diagram corresponding to remaining degrees of freedom available on the user interface with respect to the FOV over the image data displayed on the display module.
10. The surgical system of clause 9, wherein the coordinate diagram includes a first axis diagram corresponding to a first virtual axis and a second axis diagram corresponding to a second virtual axis.
11. The surgical system of clause 10, wherein the first axis diagram includes a first diagram marker associated with the current tilt along the first virtual axis and the second axis diagram includes a second diagram marker associated with the current tilt along the second virtual axis.
12. The surgical system as in clause 11, wherein the first diagram marker includes a first colorization of a segment along the first axis diagram, the length of the segment corresponding to the current tilt along the first vertical axis.
13. The surgical system as in clause 12, wherein the second diagram marker includes a second colorization of a segment along the second axis diagram, the length of the segment corresponding to the current tilt along the second virtual axis.
14. The surgical system as in one of clauses 11-13, wherein the first axis diagram includes a first diagram limit indicating the allowable remaining tilt along the first virtual axis and the second axis diagram includes a second diagram limit indicating the allowable remaining tilt along the second virtual axis.
15. The surgical system of clause 14, wherein the first diagram limit includes a terminal end of the first axis diagram and the second diagram limit includes a terminal end of the second axis diagram.
16. The surgical system as in one of clauses 11-15, wherein the coordinate diagram further includes a third axis diagram corresponding to movement of the patient operating region along a third virtual axis.
17. The surgical system as in one of clauses 9-16, wherein the coordinate diagram is selectively generated and removed from the image data.
18. A surgical system comprising:
   a user interface;
   an operating table supported on a pedestal that can selectively tilt, based on instructions from the user interface, the operating table about a longitudinal axis and a transverse axis;
   at least one robotic arm supported by the operating table and moveable relative to the operating table via the user interface;
   a camera connected to the robotic arm and configured to capture image data within a field-of-view ("FOV") of a patient operating region;
   a display module configured to display the image data; and
   a control system configured to:
      receive a current tilt of the operating table about the longitudinal axis and the transverse axis; and
      graphically generate a coordinate diagram over the image data displayed on the display module, the coordinate diagram including one or more virtual axes corresponding to a current position and degrees of freedom available on the user interface with respect to the FOV.
19. The surgical system of clause 18, wherein the user interface is, at least in part, generated on the display module and the coordinate diagram can be interfaced with to modify the current tilt of the operating table.
20. The surgical system of clause 18, wherein the user interface is configured to select the coordinate diagram and, via inputs into the user interface, modify the current tilt of the operating table.
21. The surgical system of clause 20, wherein the user interface includes a joystick.
22. The surgical system as in one of clauses 19-21, wherein the coordinate diagram includes a first axis diagram corresponding to a first virtual axis and a second axis diagram corresponding to a second virtual axis.
23. The surgical system of clause 22, wherein the first axis diagram includes a first diagram marker associated with the current tilt along the first virtual axis and the second axis diagram includes a second diagram marker associated with the current tilt along the second virtual axis.
24. The surgical system as in clause 22 or clause 23, wherein the first axis diagram includes a first modification marker indicating a desired tilt along the first virtual axis and the second axis diagram includes a second modification marker indicating a desired tilt along the second virtual axis.
25. The surgical system of clause 24, wherein the first modification marker is moveable along the first axis diagram and the second modification marker is moveable along the second axis diagram via inputs in the user interface.
26. The surgical system as in one of clauses 22-25, wherein the first diagram marker includes a first colorization of a segment along the first axis diagram, the length of the segment corresponding to the current tilt along the first virtual axis.
27. The surgical system as in clause 26, wherein the second diagram marker includes a second colorization of a segment along the second axis diagram, the length of the segment corresponding to the current tilt along the second virtual axis.
28. The surgical system as in one of clauses 22-27, wherein the first axis diagram includes a first diagram limit indicating the allowable remaining tilt along the first virtual axis and the second axis diagram includes a second diagram limit indicating the allowable remaining tilt along the second virtual axis.
29. The surgical system as in one of clauses 22-28, wherein the coordinate diagram further includes a third axis diagram corresponding to movement of the patient operating region along a third virtual axis.
30. The surgical system of clause 22, wherein the user interface is configured to selectively prevent movement about the longitudinal axis based on a selection of the first axis diagram or selectively prevent movement about the transverse axis based on a selection of the second axis diagram.
31. A computer program product comprising:
   a non-transitory computer-readable storage medium readable by one or more processing circuits and storing instructions for execution by one or more processors for performing a method of visually guiding, via a display module, a surgeon in tilting an operating table about a longitudinal axis and a transverse axis, the instructions on the non-transitory computer-readable storage medium configured to carry out steps comprising:
   receiving, from the operating table, a current tilt of the operating table about the longitudinal axis and the transverse axis; and
   graphically generating a gravity vector on the display module, the gravity vector including one or more virtual axes corresponding to degrees of freedom available on the user interface with respect to the FOV.
32. The product of clause 31, further including generating the gravity vector with a diagram of a first virtual axis, a second virtual axis, and a marking positioned relative to the first and second virtual axes that corresponds to the current tilt.
33. The product of clause 31, further including generating the marker as a vector exhibiting both direction and magnitude of the current tilt.
34. The product according to clause 31, further including generating the gravity vector with an outer boundary corresponding to an allowable amount of tilt from the FOV, the first and second virtual axes intersecting within the outer boundary.
35. The product of clause 34, wherein the generated outer boundary includes an elliptical shape.
36. A method of visually guiding, via a display module, a surgeon in tilting an operating table about a longitudinal axis and a transverse axis, the method comprising:
   receiving, from the operating table, a current tilt of the operating table about the longitudinal axis and the transverse axis; and
   graphically generating a coordinate diagram over the image data displayed on the display module, the coordinate diagram including one or more virtual axes corresponding to a current position and degrees of freedom available on the user interface with respect to the FOV.
37. The method of clause 36, further including graphically generating the coordinate diagram with a current tilt with respect to the FOV.
38. The method of clause 37, further including graphically generating the coordinate diagram with a first axis diagram of a first color corresponding to a first virtual axis and a second axis diagram of a second color that is different than the first color corresponding to a second virtual axis.
39. The method of clause 38, further including visualizing the current tilt along the first virtual axis as a first diagram marker that includes a first colorization of a segment along the first axis diagram, the length of the segment corresponding to the current tilt along the first virtual axis.
40. The method of clause 39, wherein the first colorization is generated as a brightened first color.
41. The method as in one of clauses 38-40, further including generating the current tilt along the second virtual axis as a second diagram marker that includes a second colorization of a segment along the second axis diagram, the length of the segment corresponding to the current tilt along the second virtual axis.
42. The method of clause 41, wherein the second colorization is generated as a brightened second color.
43. The method of clause 36, further including generating the first axis diagram with a first diagram limit indicating the allowable remaining tilt along the first virtual axis and the second axis diagram includes a second diagram limit indicating the allowable remaining tilt along the second virtual axis.
44. The method of clause 43, further including generating the first diagram limit with a terminal end of the first axis diagram and generating the second diagram limit with a terminal end of the second axis diagram.
45. The method as in one of clauses 38-44, further including generating a selectively implementable lock on the first axis diagram and the second axis diagram, the lock setting a limit of tilt about the first and second virtual axes.
46. The method of clause 45, wherein the lock is moveable along the first axis diagram and second axis diagram proportional to the limit of tilt.

It will be understood by one having ordinary skill in the art that construction of the described disclosure and other components is not limited to any specific material. Other exemplary embodiments of the disclosure disclosed herein may be formed from a wide variety of materials, unless described otherwise herein.

For purposes of this disclosure, the term "coupled" (in all of its forms, couple, coupling, coupled, etc.) generally means the joining of two components (electrical or mechanical) directly or indirectly to one another. Such joining may be stationary in nature or movable in nature. Such joining may be achieved with the two components (electrical or mechanical) and any additional intermediate members being integrally formed as a single unitary body with one another or with the two components. Such joining may be permanent in nature or may be removable or releasable in nature unless otherwise stated.

As used herein, the term "about" means that amounts, sizes, formulations, parameters, and other quantities and characteristics are not and need not be exact, but may be approximate and/or larger or smaller, as desired, reflecting tolerances, conversion factors, rounding off, measurement error and the like, and other factors known to those of skill in the art. When the term "about" is used in describing a value or an end-point of a range, the disclosure should be understood to include the specific value or end-point referred to. Whether or not a numerical value or end-point of a range in the specification recites "about," the numerical value or end-point of a range is intended to include two embodiments: one modified by "about," and one not modified by "about." It will be further understood that the end-points of each of the ranges are significant both in relation to the other end-point, and independently of the other end-point.

The terms "substantial," "substantially," and variations thereof as used herein are intended to note that a described feature is equal or approximately equal to a value or description. For example, a "substantially planar" surface is intended to denote a surface that is planar or approximately planar. Moreover, "substantially" is intended to denote that two values are equal or approximately equal. In some embodiments, "substantially" may denote values within about 10% of each other, such as within about 5% of each other, or within about 2% of each other.

It is also important to note that the construction and arrangement of the elements of the disclosure, as shown in the exemplary embodiments, is illustrative only. Although only a few embodiments of the present innovations have been described in detail in this disclosure, those skilled in the art who review this disclosure will readily appreciate that many modifications are possible (e.g., variations in sizes, dimensions, structures, shapes and proportions of the various elements, values of parameters, mounting arrangements, use of materials, colors, orientations, etc.) without materially departing from the novel teachings and advantages of the subject matter recited. For example, elements shown as integrally formed may be constructed of multiple parts, or elements shown as multiple parts may be integrally formed, the operation of the interfaces may be reversed or otherwise varied, the length or width of the structures and/or members or connector or other elements of the system may be varied, the nature or number of adjustment positions provided between the elements may be varied. It should be noted that the elements and/or assemblies of the system may be constructed from any of a wide variety of materials that provide sufficient strength or durability, in any of a wide variety of colors, textures, and combinations. Accordingly, all such modifications are intended to be included within the scope of the present innovations. Other substitutions, modifications, changes, and omissions may be made in the design, operating conditions, and arrangement of the desired and other exemplary embodiments without departing from the spirit of the present innovations.

It will be understood that any described processes or steps within described processes may be combined with other disclosed processes or steps to form structures within the scope of the present disclosure. The exemplary structures and processes disclosed herein are for illustrative purposes and are not to be construed as limiting.

## Claims

1. A surgical system (10) comprising:
a user interface (14);
an operating table (16) supported on a pedestal (20) that can selectively tilt, based on instructions from the user interface (14), the operating table (16) about a longitudinal axis L(a) and a transverse axis T(a);
at least one robotic arm (18) supported by the operating table (16) and moveable relative to the operating table (16) via the user interface (14);
a camera (21) connected to the robotic arm (18) and configured to capture image data (22) within a field-of-view ("FOV") of a patient operating region (24);
a display module (26) configured to display the image data (22); and
a control system (100) configured to:
receive a current tilt of the operating table (16) about the longitudinal axis L(a) and the transverse axis T(a); and
graphically generate a gravity vector (28) over the image data (22) displayed on the display module (26), the gravity vector (28) including one or more virtual axes V(La), V(Ta) corresponding to degrees of freedom available on the user interface (14) with respect to the FOV.

2. The surgical system (10) of claim 1, wherein the one or more virtual axes V(La), V(Ta) include a diagram of a virtual representation of controller movement in the first virtual axis V(La) and a virtual representation of controller movement in the second virtual axis V(Ta).

3. The surgical system (10) of claim 2, wherein the gravity vector (28) further includes a marking (50) positioned relative to the first and second virtual axes V(La), V(Ta) that corresponds to the current forces of gravity with respect to the FOV.

4. The surgical system (10) of claim 3, wherein the marking (50) includes an arrow as a vector exhibiting both direction and magnitude of the current tilt.

5. The surgical system (10) as in one of claims 1-4, wherein the gravity vector (28) includes an outer boundary (52) corresponding to an allowable amount of tilt.

6. The surgical system (10) as in one of claims 1-4, wherein the user interface (14) is, at least in part, generated on the display module (26) and the gravity vector (28) can be interfaced with to modify the current tilt of the operating table (16).

7. The surgical system (10) of claim 1, wherein the control system (100) is further configured to:
graphically generate a coordinate diagram (30) corresponding to remaining degrees of freedom available on the user interface (14) with respect to the FOV over the image data (22) displayed on the display module (26).

8. The surgical system (10) of claim 7, wherein the coordinate diagram (30) includes a first axis diagram (54) corresponding to a first virtual axis V(La) and a second axis diagram (56) corresponding to a second virtual axis V(Ta).

9. The surgical system (10) of claim 8, wherein the first axis diagram (54) includes a first diagram marker (60) associated with the current tilt along the first virtual axis V(La) and the second axis diagram (56) includes a second diagram marker (62) associated with the current tilt along the second virtual axis V(Ta).

10. The surgical system (10) as in claim 9, wherein the first diagram marker (60) includes a first colorization of a segment along the first axis diagram (54), the length of the segment corresponding to the current tilt along the first vertical axis.

11. The surgical system (10) as in claim 9 or claim 10, wherein the second diagram marker (62) includes a second colorization of a segment along the second axis diagram (56), the length of the segment corresponding to the current tilt along the second virtual axis V(Ta).

12. The surgical system (10) of claim 11, wherein the first axis diagram (54) includes a first diagram limit (68) indicating the allowable remaining tilt along the first virtual axis V(La) and the second axis diagram (56) includes a second diagram limit (70) indicating the allowable remaining tilt along the second virtual axis V(Ta).

13. The surgical system (10) of claim 12, wherein the first diagram limit (68) includes a terminal end of the first axis diagram (54) and the second diagram limit (70) includes a terminal end of the second axis diagram (56).

14. The surgical system (10) of claim 9 or claim 10, wherein the coordinate diagram (30) further includes a third axis diagram (58) corresponding to movement of the patient operating region (24) along a third virtual axis V(Va).

15. The surgical system (10) of claim 9 or claim 10, wherein the coordinate diagram (30) is selectively generated and removed from the image data (22).
